Europäisches Patentamt

European Patent Office

Office européen des brevets

(11) Veröffentlichungsnummer: **0 345 504**
**A1**

## (12) EUROPÄISCHE PATENTANMELDUNG

(21) Anmeldenummer: **89108988.0**

(22) Anmeldetag: **19.05.89**

(51) Int. Cl.4: **B01J 8/04 , C01C 1/04 , C07C 29/15**

(30) Priorität: **08.06.88 DE 3819453**

(43) Veröffentlichungstag der Anmeldung:
**13.12.89 Patentblatt 89/50**

(84) Benannte Vertragsstaaten:
**AT BE CH DE ES FR GB IT LI NL**

(71) Anmelder: **Uhde GmbH**
**Friedrich-Uhde-Strasse 15 Postfach 262**
**D-4600 Dortmund 1(DE)**

(72) Erfinder: **Weicken, Reimund, Dipl.-Ing.**
**Hinterm Südfriedhof 21**
**D-4750 Unna(DE)**

(54) **Vorrichtung zur Durchführung exothermer, katalytischer Gasreaktionen für die Ammoniak- oder Methanol-Synthese.**

(57) Bei einer Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatz mindestens zwei übereinander angeordnete Katalysatorbehälter (6,10) in ringförmiger zylindrischer Anordnung mit gasdurchlässiger Innen- und Außenwand für radiale Gasströmung von außen nach innen und zwei Gas/Gas-Wärmetauscher (5,9) in Rohrbündelbauart zentral innerhalb des ersten und des zweiten Katalysatorbehälters enthält, soll eine Lösung geschaffen werden, wobei auf ein zentrales Leitrohr verzichtet werden kann.

Dies wird dadurch erreicht, daß der erste Wärmetauscher (5) rohrseitig mit dem inneren Ringraum (11) des zweiten Katalysatorbettes (6) und mit dem Auslaßstutzen (15) für das Reaktionsgas verbunden ist, daß der erste Wärmetauscher (5) für das aufzuheizende Frischgas mantelseitig verbunden ist mit dem unteren Freiraum (4) und der Verbindungskammer (7), daß der Rohrboden (13) im äußeren Bereich Öffnungen (12) aufweist als Verbindung zwischen Ringraum (11) und der oberen Rohrbodenkammer (14).

## Vorrichtung zur Durchführung exothermer, katalytischer Gasreaktionen für die Ammoniak- oder Methanol-Synthese

Die Erfindung betrifft eine Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatzmantel mindestens zwei übereinander angeordnete Katalysatorbehälter in ringförmiger zylindrischer Anordnung mit gasdurchlässiger Innen- und Außenwand für radiale Gasströmung von außen nach innen und zwei Gas/Gas-Wärmetauscher in Rohrbündelbauart zentral innerhalb des ersten und des zweiten Katalysatorbehälters enthält und wobei der zweite Gas/Gas-Wärmetauscher rohrseitig vom Frischgas und mantelseitig vom Reaktionsgas durchströmt wird.

Die katalytische Hochdrucksynthese zur Erzeugung von Ammoniak und Methanol ist ein exothermer Prozeß. Man ist seit jeher bestrebt, diesen Prozeß bei solchen Temperaturen ablaufen zu lassen, daß die thermischen Verhältnisse zu optimaler Reaktion, d.h. zu optimalem Umsatz, führen. Die Einhaltung der günstigsten Reaktionstemperaturen geschieht durch Abkühlung der bei der Reaktion sich stark erwärmenden Reaktionsgase. Da das Frischgas mit einer Temperatur von über 300 °C auf die 1. Katalysatorschicht gegeben werden muß, wird angestrebt, das Frischgas mit dem heißen Reaktionsgas in indirektem Wärmetausch aufzuheizen.

Die Abkühlung des Reaktionsgases kann nun entweder so erfolgen, daß die Reaktion fast isothermisch verläuft wie es bei Katalysatorröhrenöfen oder Vollraumöfen mit Kühlschlangen in der Katalysatormasse der Fall ist, oder so, daß die Abkühlung stufenweise nach einzelnen Katalysatorpackungen erfolgt, in die die gesamte Katalysatormasse aufgeteilt ist.

Es sind zahlreiche Vorrichtungen bzw. Ofentypen bekannt zur Lösung der o.g. Forderungen. Es ist beispielsweise bekannt, die Katalysatormasse in mehreren Lagen untereinander anzuordnen und in den Zwischenräumen Röhren-Wärmetauscher für aufzuheizendes Frischgas einzubauen, um die Reaktionstemperaturen zu senken.

Nach DE-OS 33 43 114 ist ein Radial-Reaktor mit zentral angeordneten Zwischenwärmetauschern bekannt. Die beiden Zwischenwärmetauscher weisen in etwa gleiche Außendurchmesser auf und ihre, mit geringem Abstand zueinander liegenden beiden inneren Rohrböden, sind mittels eines Mantelbleches gasdicht verbunden. Über ein zentrales Leitrohr besteht eine durchgängige Verbindung vom Freiraum zwischen Hochdruckmantel und Einsatz zum unteren Rohrboden des unteren Wärmetauschers. Dieses zentrale Leitrohr benötigt Hochdruckraum.

Der Erfindung liegt die Aufgabe zugrunde, auf das zentrale Leitrohr zu verzichten.

Die Aufgabe wird erfindungsgemäß gelöst durch eine Vorrichtung zur Durchführung exothermer, katalytischer Gasreaktionen für die Ammoniak- oder Methanol-Synthese mit den Merkmalen entsprechend den konstruktiven Ausgestaltungen gemäß den Kennzeichen des Hauptanspruches.

Nach einer Ausgestaltung der Erfindung kann der zweite Wärmetauscher auch eine Zufuhrleitung für Zumischgas zum aufzuheizenden Frischgas enthalten.

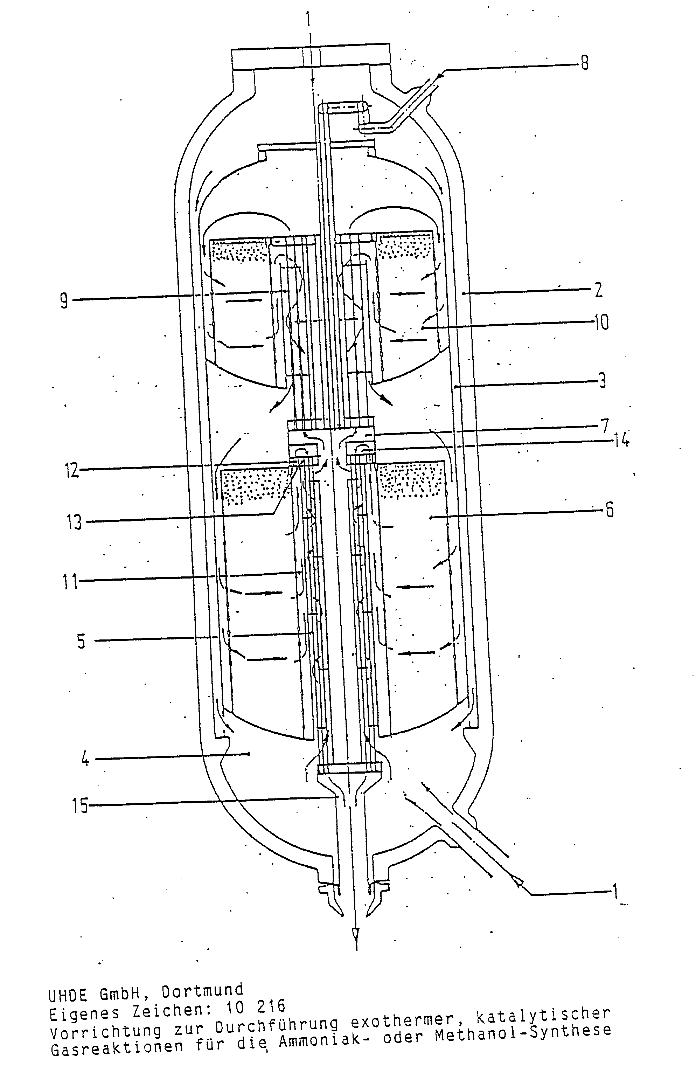

Die nachfolgend anhand der Abbildung ausführlich beschriebene erfindungsgemäße Vorrichtung erlaubt einen optimalen Ausnutzungsgrad des Hochdruckraumes mittels Wärmetauscherfläche.

Ein Ausführungsbeispiel der Erfindung ist in der Zeichnung dargestellt und wird im folgenden näher beschrieben.

Der Hauptgasstrom 1 tritt oben oder unten in den Druckmantel 2 ein und strömt über den Ringspalt 3, den Mantel kühlend, bzw. unten über den Freiraum 4 direkt in den unteren Wärmetauscher 5 um die Rohre ein. Bei Durchströmung des unteren Wärmetauschers 5 um die Rohre wird es durch Reaktionsgas aus dem zweiten Katalysatorbett 6 aufgeheizt. Dann tritt es in eine Verbindungskammer 7 ein, in der kaltes Bypaßgas 8 zur Temperaturkontrolle beigemischt werden kann und wird nachfolgend durch die Rohre des oberen Wärmetauschers 9 strömend auf die Eintrittstemperatur des ersten Bettes 10 aufgeheizt. Das erste Katalysatorbett 10 wird radial von außen nach innen durchströmt und das heiße Reaktionsgas nach oben zum Eintritt in den Mantelraum des oberen Wärmetauschers 9 geleitet. Mantelseitig nach unten strömend kühlt es auf die Eintrittstemperatur des zweiten Bettes ab. Dieses Katalysatorbett 6 wird ebenfalls radial von außen nach innen durchströmt. Das heiße Reaktionsgas tritt dann nach oben aus dem inneren Ringraum 11 über Öffnungen 12 im äußeren Bereich des Rohrbodens 13 in die obere Rohrbodenkammer 14 des nun rohrseitig durchströmten, unteren Wärmetauschers 5. Nach Kühlung des rohrseitig strömenden Gases wird dieses im Auslaßstutzen 15 gesammelt und zentral aus dem Hochdruckgefäß herausgeführt.

Die Vorteile dieser Strömungsführung liegen in der Vermeidung eines zentralen Gasleitrohres und der Möglichkeit, mit dem Hauptgasstrom sowohl oben als auch unten in den Konverter eintreten zu

können.

**Ansprüche**

1. Vorrichtung zur Durchführung exothermer, katalytischer Gasreaktionen für die Ammoniak- oder Methanol-Synthese, im wesentlichen bestehend aus einem Hochdruckmantel und einem Einsatz mit oberem Abschlußdeckel, wobei der Einsatz mindestens zwei übereinander angeordnete Katalysatorbehälter (6,10) in ringförmiger zylindrischer Anordnung mit gasdurchlässiger Innen-und Außenwand für radiale Gasströmung von außen nach innen und zwei Gas/Gas-Wärmetauscher (5,9) in Rohrbündelbauart zentral innerhalb des ersten und des zweiten Katalysatorbehälters enthält, wobei der zweite Gas/Gas-Wärmetauscher (19) rohrseitig vom Frischgas und mantelseitig vom Reaktionsgas durchströmt wird,
dadurch gekennzeichnet, daß der erste Wärmetauscher (5) rohrseitig mit dem inneren Ringraum (11) des zweiten Katalysatorbettes (6) und mit dem Auslaßstutzen (15) für das Reaktionsgas verbunden ist, daß der erste Wärmetauscher (5) für das aufzuheizende Frischgas mantelseitig verbunden ist mit dem unteren Freiraum (4) und der Verbindungskammer (7), daß der Rohrboden (13) im äußeren Bereich Öffnungen (12) aufweist als Verbindung zwischen Ringraum (11) und der oberen Rohrbodenkammer (14).

2. Vorrichtung nach Anspruch 1, gekennzeichnet durch eine Zuführleitung (8) für Zumischgas, die durch den zweiten Gas/Gas-Wärmetauscher (9) bis in die Verbindungskammer (7) führt.

UHDE GmbH, Dortmund
Eigenes Zeichen: 10 216
Vorrichtung zur Durchführung exothermer, katalytischer
Gasreaktionen für die Ammoniak- oder Methanol-Synthese

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 143 280  (UHDE GmbH)<br>* Seite 4, Absatz 4; Seite 5, Absatz 1;<br>Seite 6, Absatz 1 *<br>--- | 1,2 | B 01 J    8/04<br>C 01 C    1/04<br>C 07 C   29/15 |
| A | EP-A-0 222 069  (AMMONIA CASALE S.A.)<br>* Seite 3, Absätze 2,3 *<br>----- | 1 | |
| | | | **RECHERCHIERTE SACHGEBIETE (Int. Cl.4)**<br><br>B 01 J<br>C 01 C |

Der vorliegende Recherchenbericht wurde für alle Patentansprüche erstellt

| Recherchenort | Abschlußdatum der Recherche | Prüfer |
|---|---|---|
| DEN HAAG | 02-08-1989 | KERRES P.M.G. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)